# EUROPEAN PATENT APPLICATION

(11) **EP 2 340 818 A1**
(43) Date of publication of application: **06.07.2011**
(21) Application number: 09384007.2
(22) Date of filing: 23.12.2009
(51) Int. Cl.: A61K 31/137, A61K 31/415

(54) **Co-crystals of venlafaxine and celecoxib**

(71) Applicant: Laboratorios del. Dr. Esteve, S.A., 08041 Barcelona (ES)
(72) Inventor: Plata Salaman, Carlos Ramon, 08950 Esplugues de Llobregat, ( Barcelona ) (ES); Videla Cés, Sebastià, 08017 Barcelona (ES); Tesson, Nicolas,, 08902 L'hospitalet de Llobregat (ES); Trilla Castano, Montserrat, 08013 Barcelona (ES)
(74) Representative: Peters, Hajo

(57) **Abstract**

The present invention relates to a co-crystal of celecoxib and venlafaxine, processes for preparation of the same and its use as medicaments or in pharmaceutical formulations, more particularly for the treatment of pain, including chronic pain; or of depression in patients which suffer from chronic pain and/or chronic inflammation or in patients with a chronic musculo-skeletal inflammatory illness, with the inflammatory illness preferably being selected from osteoarthritis or rheumatoid arthritis.

## Description

The present invention relates to co-crystals of venlafaxine and celecoxib, processes for preparation of the same and their uses as medicaments or in pharmaceutical formulations, more particularly for the treatment of pain, including chronic pain; or of depression in patients which suffer from chronic pain and/or chronic inflammation or in patients with a chronic musculo-skeletal inflammatory illness, with the inflammatory illness preferably being selected from osteoarthritis or rheumatoid arthritis.

**Pain and depression.** Psychological comorbidity is an important and frequent complication significantly changing the prognosis and course of chronic pain (Tunks et al., 2008 [1]; Kojima et al., 2009 [2]; Campbell et al., 2003 [3]). In some research articles it has been suggested that 40%-50% of the patients suffering from chronic pain suffer also from depressive disorders. Furthermore, higher levels of depression have been found to be related to a higher likelihood of experiencing pain, higher pain severity, more frequent pain and to a premature termination from pain rehabilitation programs (Banks, & Kerns, 1996 [4]; Gatchel et al., 2007 [5]). In fact, responses to chronic pain and depression appear so similar that they are often confused by medical professionals (MacDonald & Leary, 2005 [6]). This association between chronic pain and depression is not surprising since serotonin and norepinephrine, the neurotransmitters most associated with depression, play also key roles in the modulation of pain (Millan, 1999 [7]; Arnold et al., 2008 [8]).

Different chronic musculo-skeletal inflammatory Illnesses, like Rheumatoid arthritis (RA), Osteoarthritis (OA), are one of the most common reasons for seeking medical attention, sickness absence from work and long term disability. Patients suffering from this kind of illness not only suffer from the consequences of a chronic inflammation but also ― sometimes increasingly over time ― suffer from chronic pain symptoms.

RA is a chronic, systemic inflammatory disorder that may affect many tissues and organs, but principally attacks the joints producing an inflammatory synovitis that often progresses to destruction of the articular cartilage and ankylosis of the joints. The cause of rheumatoid arthritis is unknown; but the autoimmunity plays a pivotal role in its chronicity and progression. About 1% of the world's population is affected by rheumatoid arthritis, women three times more often than men. Onset is most frequent between the ages of 40 and 50.

OA is a flaring degenerative arthropathy and a disorder which can potentially affect all synovial joints. OA is characterised by degeneration and regeneration of articular cartilage and bone. The pathological changes can be focal or more generalised and these changes often correlate poorly at one time point with clinical signs and symptoms but correlate longitudinally.

It is rare for OA to develop before the age of 40, but after this age the incidence increases, especially in women. OA of the hip may start a decade later than OA of the knee or hand. The prevalence of symptomatic knee OA in patients aged 35-54 years is around 1%, whereas about 40% of the population aged over 65 has symptomatic OA of the knee or hip. OA of the knee is more prevalent than hip OA.

The above mentioned inflammatory illnesses, OA and RA, can be a disabling and painful condition, which can lead to substantial loss of functioning and mobility. In fact, depression is also associated with increased functional disability in these patients. Long-term studies have shown that depression occurs following deterioration in functional ability, particularly with regard to activities which an individual regards as being important, e.g. visiting the family, going away on holiday, etc. while also the pain experienced may contribute to the overall psychological condition. For example, RA patients are twice as likely to suffer from depression as members of the general population. In RA patients, depression not only contributes its own additional burden but also interacts with the way patients perceive and cope with their physical illness and how they interact with their rheumatologist and general practitioner. Thus, depression increases the burden of RA to the patient and society.

Pain is a complex response that has been functionally categorized into sensory, autonomic, motor, and affective components. The sensory aspect includes information about stimulus location and intensity while the adaptive component may be considered to be the activation of endogenous pain modulation and motor planning for escape responses. The affective component appears to include evaluation of pain unpleasantness and stimulus threat as well as negative emotions triggered by memory and context of the painful stimulus.

In general, pain conditions can be divided into chronic and acute. Chronic pain includes neuropathic pain and chronic inflammatory pain, for example arthritis, or pain of unknown origin, as fibromyalgia. Acute pain usually follows non-neural tissue injury, for example tissue damage from surgery or inflammation, or migraine.

Multi-Mechanism Treatment Approach. Accordingly, evidence-based recommendations for the treatment of pain recommend paying particular attention to identify coexisting depression, anxiety, sleep disturbances, and other adverse impacts of pain on health-related quality of life and that both pain and its adverse effects should be reassessed frequently. The treatment of chronic pain and related depression is a challenging healthcare problem and targeting medications from various classes are necessary to decrease pain, improve mood, and restore normal sleep. The available drugs used as mono-treatment to treat both pain and depression syndromes have incomplete efficacy and dose-limiting adverse effects.

WO2004/060366 describes a method for the treatment of a CNS disorder, pain and inflammation in a subject in need of such treatment comprising administering to the subject a compound selected from the group consisting of duloxetine, venlafaxine and atomoxetine and a long list of COX-2 inhibitors. While trying to approach the treatment of CNS disorders like depression encountered in a patient together with inflammatory or pain symptoms the offered suggestion is a co-treatment with at least two different active agents covering a wide range of many possible combinations. On one hand such a co-treatment is often very inconvenient for the patient and on the other hand WO2004/060366 is also silent on combinations that get rid of the extra and superfluous elements that are usually seen in a standard combination treatment with well-known standard active agents (as opposed to that would be offered by a selective co-crystals).

The objective of the invention was to provide new pharmacological means for the treatment of depression in connection with chronic diseases - like those with inflammatory and painful syndromes - by choosing a highly suitable combination of active compounds, while preferably also improving the properties of these active compounds and thus providing a new well-drugable form comprising the highly suitable combination of the active compounds.

Also a big proportion of the patients with chronic musculo-skeletal inflammatory illnesses and depression are being co-treated with anti-inflammatory/painkillers and antidepressants remedies which is often very inconvenient for the patient. Thus it was further objective of the current invention to provide a new drug combining these activities in one form for treating both conditions at the same time.

Clinical improvements/advantages desirable to the new drugable form would include any single one of the following or even more preferable even more than one of the following advantages below:
● Being most effective through different analgesic mechanisms providing more effective pain relief for a broader spectrum of pain.
● Reducing adverse drug reactions.
● Reducing adverse drug reactions while having an at least additive effect of the active compounds in the new drugable form.
● Showing synergistic activity regarding pain and or depression.
● Providing analgesia more quickly by combining a medication with a rapid onset of effect compared to the art that most often requires several weeks of treatment before maximum benefit is achieved.
● Allowing optimization of the clinical management of depressive patients with an associated chronic pain condition.
● Optimizing treatment for chronic pain patients.
● Allowing treatment of patients with ongoing chronic pain and affective distress for whom traditional conservative treatments have failed.
● Enhancing the treatment adherence or compliance of patients who suffer from depression associated to chronic pain.

This objective was achieved by providing a new co-crystal of celecoxib and venlafaxine. Even though, it was well-known that attempts to obtain co-crystals of certain molecules often encounter a huge number of chemical difficulties to be overcome ― from which most analgesics or antidepressants are no exception - it was, surprisingly, found that celecoxib and venlafaxine as starting elements are suitable to form co-crystals. Such co-crystals on one hand are very effective in the treatment of depression in connection with chronic diseases - like those with inflammatory and painful syndromes ― but on the other hand also show improved properties if compared to celecoxib or venlafaxine alone or to the (physical) mixture of celecoxib and venlafaxine.

Thus, the present invention covers a co-crystal comprising venlafaxine either as a free base or as its physiologically acceptable salt and celecoxib

Celecoxib is an anti-inflammatory and pain killer drug and it is one of the most used treatments for chronic musculo-skeletal inflammatory illnesses. Celecoxib, 4-[5-(4-methylphenyl)-3-(trifluoromethyl)pyrazol-1-yl]benzenesulfonamide has the following formula: 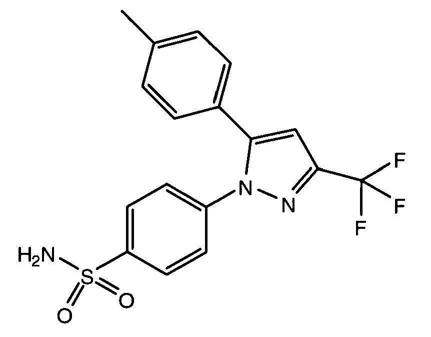 Celecoxib is an oral, highly selective cyclooxygenase-2 (COX-2) inhibitor, and it is indicated for the treatment of symptomatic relief in the treatment of osteoarthritis, rheumatoid arthritis and ankylosing spondylitis (Goldenber, 1999 [9]). This high selectivity allows celecoxib and other COX-2 inhibitors to reduce inflammation (and pain) while minimizing gastrointestinal adverse drug reactions (e.g. stomach ulcers) that are common with non-selective NSAIDs.

Cycloxygenase is responsible for the generation of prostaglandins. Two isoforms, COX-1 and COX-2, have been identified. COX-2 is the isoform of the enzyme that has been shown to be induced by pro-inflammatory stimuli and has been postulated to be primarily responsible for the synthesis of prostanoid mediators of pain, inflammation, and fever. COX-2 is also involved in ovulation, implantation and closure of the ductus arteriosus, regulation of renal function, and central nervous system functions (fever induction, pain perception and cognitive function). It may also play a role in ulcer healing. COX-2 has been identified in tissue around gastric ulcers in man but its relevance to ulcer healing has not been established. Celecoxib inhibits COX-2 without affecting COX-1. COX-1 is involved in synthesis of prostaglandins and thromboxane, but COX-2 is only involved in the synthesis of prostaglandin. Therefore, inhibition of COX-2 inhibits only prostaglandin synthesis without affecting thromboxane and thus has no effect on platelet aggregation or blood clotting

Venlafaxine is an antidepressant drug and it is indicated for the treatment of major depressive disorder including depression accompanied by anxiety. Venlafaxine, (*rac*)-1-[2-dimethylamino-1-(4-methoxyphenyl)-ethyl]cyclohexanol, has the following formula:

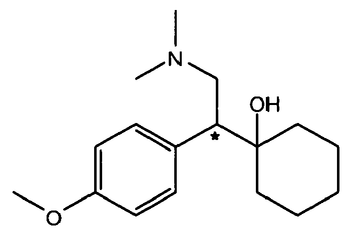

Its mechanism of action in humans is believed to be associated with its potentiation of neurotransmitter activity in the central nervous system. Preclinical studies have shown that venlafaxine and its major metabolite, O-desmethylvenlafaxine, are potent neuronal serotonin and noradrenaline re-uptake inhibitors (SNRI) and weak inhibitors of dopamine reuptake. In addition, venlafaxine and O-desmethylvenlafaxine reduce β-adrenergic responsiveness in animals after both acute (single dose) and chronic administration. Venlafaxine and its major metabolite appear to be equipotent with respect to their overall action on neurotransmitter re-uptake. Venlafaxine does contain a chiral C-atom (marked with a star) and thus may take the form of a racemate (rac)-venlafaxine or of an enantiomer ((+)-venlafaxine or (-)-venlafaxine) or mixtures thereof.

Celecoxib and venlafaxine, both have their own disadvantages when used alone or in the form of the salts known from the art. Thus, for example celecoxib is only slightly soluble in water and this is severely limiting its use in pharmaceutical formulations. On the other hand venlafaxine has many side effects. The main side effects when using venlafaxine include: anxiety; blurred vision; changes in taste; constipation; decreased sexual desire or ability; dizziness; drowsiness; dry mouth; flushing; headache; increased sweating; loss of appetite; nausea; nervousness; stomach upset; trouble sleeping; vomiting; weakness; weight loss; yawning.

As said above, the new co-crystals show many advantages.

Thus, on one hand the physico-chemical properties are improved. Any attempt at formulating a medicament or pharmaceutical formulation comprising the co-crystals shows that is much easier to handle these active agents with just one solid to manipulate opposed to the art, and also seem to show an enhanced stability of these pharmaceutical formulations. In addition the solubility of at least one of the active compounds (e.g. celecoxib) seems to be enhanced.

In addition the combination of two active principles into one unique species (liker a co-crystal) seems to allow for a better Pharmacokinetic/Pharmacodynamic (PKPD)

On the other hand, also in the treatment situation the co-crystals according to the invention seem to show many advantages. Thus, under the proper circumstance this new solid drugable form is possibly achieving some modulation of the pharmacological effects, while also enhancing the patient's compliance.

Especially, at first impressions the new drugable form seems to be capable of fulfilling also one or even some of the desirable clinical advantages listed above, like
● providing more effective pain relief for a broader spectrum of pain,
● reducing adverse drug reactions,
● reducing side effects with an additive effect of the compounds in the co-crystal,
● showing synergistic activity regarding pain and or depression,
● providing analgesia more quickly with a rapid onset of effect,
● allowing treatment of patients with ongoing chronic pain and affective distress for whom traditional conservative treatments have failed, or
● enhancing the treatment adherence or compliance of patients who suffer from depression associated to chronic pain, or
● having therapeutically activity while having a subactive dose of each active principle of the co-crystal.

In addition, it seems that in clinical practice the co-administration of venlafaxine and celecoxib (especially in form of the co-crystal according to the invention) would be especially useful for the often coexisting disorders depression and chronic pain, with both compounds combined in the co-crystal showing both their analgesic and their antidepressant properties.

As said above, the present invention covers a co-crystal comprising venlafaxine either as a free base or as its physiologically acceptable salt and celecoxib or a co-crystal of venlafaxine either as a free base or as its physiologically acceptable salt and celecoxib either in neutral form or as its physiologically acceptable salt.

"Co-crystal" as used herein is defined as a crystalline material comprising two or more compounds at ambient temperature (20 to 25°C, preferably 20°C), of which at least two are held together by weak interaction, wherein at least one of the compounds is a co-crystal former. Weak interaction is being defined as an interaction which is neither ionic nor covalent and includes for example: hydrogen bonds, van der Waals forces, and π―π interactions. Solvates of venlafaxine that do not further comprise celecoxib are not co-crystals according to the present invention. The co-crystals may however, include one or more solvate molecules in the crystalline lattice. Just for the sake of clarity the distinction between crystalline salt and a co-crystal has to be stressed here. An API bound to another compound forming a salt by means of ionic interaction can be considered as one "compound" according to the invention, but it cannot be considered as two compounds by itself.

In scientific literature there currently is some discussion on the proper use of the word co-crystal (see for example Desiraju, CrystEngComm, 2003, 5(82), 466-467 and Dunitz, CrystEngComm, 2003, 5(91), 506). A recent article by Zaworotko (Zaworotko, Crystal Growth & Design, Vol. 7, No. 1, 2007, 4-9) gives a definition of co-crystal which is in line with the definition given above.

"Pain" and especially "chronic pain" is defined by the International Association for the Study of Pain (IASP) as "an unpleasant sensory and emotional experience associated with actual or potential tissue damage, or described in terms of such damage (IASP, Classification of chronic pain, 2nd Edition, IASP Press (2002), 210).

"Depression" is defined as impairment of affectivity with depressive episodes.

In a further embodiment of the co-crystal according to the invention the celecoxib is in neutral form.

As celecoxib is weakly acidic with a pKa of 11.1 its "neutral form" according to the invention is defined therefore as the form in which celecoxib is free (not in form of a salt) but is ― depending on the pH ― neutral or carrying a load.

In a further embodiment of the co-crystal according to the invention the venlafaxine comprised in the co-crystal is selected from (rac)-venlafaxine, (-)-venlafaxine or (+)-venlafaxine, preferably is (rac)-venlafaxine.

As illustrated in more detail below venlafaxine as its racemate and its enantiomers (+)-venlafaxine and (-)-venlafaxine form co-crystals with celecoxib. Generally the co-crystals obtained have a specific stoichiometry.

In a further embodiment of the co-crystal according to the invention the molecular ratio between celecoxib and venlafaxine is between 3:1 and 1:3, preferably is between 2:1 and 1:2, and most preferably is 1:1.

In a further embodiment of the co-crystal according to the invention the co-crystal showing such attributes that if compared to either venlafaxine alone or to a comparable mixture of venlafaxine and celecoxib
● the solubility of the co-crystal is increased; and/or
● the dose response of the co-crystal is increased; and/or
● the efficacy of the co-crystal is increased; and/or
● the dissolution of the co-crystal is increased; and/or
● the bioavailability of the co-crystal is increased; and/or
● the stability of the co-crystal is increased; and/or
● the hygroscopicity of the co-crystal is decreased; and/or
● the form diversity of the co-crystal is decreased; and/or
● the morphology of the co-crystal is modulated.

This applies for example also to the choice of ratio between venlafaxine (as free base or salt and either as racemate or enantiomer) and celecoxib being chosen in such a way that it fulfils one or more of the above advantages if compared to either venlafaxine alone or to a comparable mixture of venlafaxine and celecoxib.

"Mixture of venlafaxine and celecoxib" is defined as a mixture of celecoxib with venlafaxine which is only a physical mixture without any coupling forces between the compounds and thus neither includes salts nor another co-crystal.

The term "salt" is to be understood as meaning any form of venlafaxine in which it assumes an ionic form or is charged and is coupled with a counter-ion (a cation) or is in solution. By this are also to be understood complexes of venlafaxine or celecoxib with other molecules and ions, in particular complexes which are complexed via ionic interactions. This also includes physiologically acceptable salt.

The term "solvate" according to this invention is to be understood as meaning any form of the venlafaxine or celecoxib in which the compound has attached to it via non-covalent binding a solvent (most likely a polar solvent) especially including hydrates and alcoholates, e.g. monohydrate.

A very preferred embodiment of the invention relates to a co-crystal according to the invention comprising celecoxib and (*rac*)-venlafaxine, and even more preferred to a co-crystal according to the invention, wherein the molecular ratio between celecoxib and *(rac)-*venlafaxine is 1:1.

In a preferred embodiment the co-crystal with a molecular ratio between celecoxib and *(rac)-*venlafaxine being 1:1 according to the invention shows a Fourier Transform Infra Red pattern with absorption bands at 3293.8 (m), 2945.7 (m), 2860.9 (m), 2830.3 (m), 1610.3 (m), 1512.5 (s), 1470.0 (s), 1410.0 (m), 1375.0 (m), 1350.3 (m), 1339.7 (s), 1238.3 (s), 1163.1 (s), 1112.1 (m), 1093.5 (m), 1039.8 (m), 974.9 (m), 840.5 (s), 825.4 (m), 805.8 (m) 615.1 (m), 544.8 (m) cm⁻¹ .

In a preferred embodiment the co-crystal with a molecular ratio between celecoxib and *(rac)-*venlafaxine being 1:1 according to the invention has a monoclinic unit cell with the following dimensions:
a = 17.74(18) Å
b = 10.17(10) Å
c = 20.11(2) Å
β= 111.20(2)°.

In a preferred embodiment the co-crystal with a molecular ratio between celecoxib and (rac)-venlafaxine being 1:1 according to the invention the endothermic sharp peak of the co-crystal corresponding to the melting point has an onset at 111 °C or preferably at 111.24°C.

In a preferred embodiment the co-crystal with a molecular ratio between celecoxib and (rac)-venlafaxine being 1:1 according to the invention the co-crystal shows an X-Ray powder diffraction pattern with peaks [2θ] at 4.73, 12.01, 14.33, 15.81, 17.43, 18.03, 18.87, 20.49, 20.65, 21.45, 22.51, 23.66, 24.94, and 26.56. In a related preferred embodiment the co-crystal with a molecular ratio between celecoxib and (rac)-venlafaxine being 1:1 according to the invention shows a X-Ray powder diffraction pattern peaks [2θ] at 4.73, 9.38, 10.18, 10.69, 12.01, 12.81, 14.33, 15.13, 15.81, 16.64, 17.43, 18.03, 18.34, 18.87, 19.63, 20.06, 20.49, 20.65, 20.87, 21.45, 21.93, 22.51, 23.19, 23.66, 24.94, 25.34, 25.83, 26.56, 27.99, 28.33, 29.87, 30.72, 31.11, 31.90, 32.24, 33.66, 34.11, 34.57, 36.56, 36.90, 37.90 and 38.35. The 2θ values were obtained using copper radiation (Cu_{Kα1} 1.54060Å).

Another embodiment of the present invention relates to a process for the production of a co-crystal according to the invention as described above comprising the steps of:
(a) adding venlafaxine and celecoxib to a container;
(b) adding a first organic solvent;
(c) optionally adding a second organic solvent;
(d) optionally removing the first organic solvent;
(e) optionally filtering-off the resulting co-crystals
(f) optionally drying the resulting co-crystals at ambient temperature under vacuum.

"Ambient temperature" (also known as "room temperature") is defined here as a temperature between 20 and 25°C, preferably being 20°C.

In a preferable embodiment the first organic solvent in this process is selected from methyl isobutyl ketone, acetonitrile, or ethanol.

In a preferable embodiment the second organic solvent in the process according to the invention is heptane.

The parts of the co-crystal according to the invention are well-known drugs. Due to this, a further object of the present invention is a medicament comprising a co-crystal according to the invention and optionally one or more pharmaceutically acceptable excipients.

Thus the invention also concerns a medicament comprising at least one co-crystal according to the invention as described above.

Thus the invention also concerns a medicament comprising at least one co-crystal according to the invention as described above and optionally one or more pharmaceutically acceptable excipients.

The invention also relates to a pharmaceutical composition comprising a therapeutically effective amount of the co-crystal according to the invention in a physiologically acceptable medium.

The invention also relates to a pharmaceutical composition comprising the co-crystal according to the invention and optionally one or more pharmaceutically acceptable excipients.

The invention also relates to a pharmaceutical composition comprising a therapeutically effective amount of the co-crystal according to the invention and optionally one or more pharmaceutically acceptable excipients.

The invention also concerns a medicament or pharmaceutical composition comprising (preferably a therapeutically effective amount of) at least one co-crystal according to the invention as described above and optionally one or more pharmaceutically acceptable excipients for the treatment of pain, including chronic pain; or of depression including depression accompanying chronic pain and/or chronic inflammation especially for the treatment of depression in patients with a chronic musculo-skeletal inflammatory illness, with the inflammatory illness preferably being selected from osteoarthritis or rheumatoid arthritis. The pain mentioned above may be chronic pain, but also severe to moderate pain.

In general, in most embodiments in which the co-crystals of venlafaxine are used (e.g. for the treatment of depression or pain (chronic pain) or depression in patients with a chronic musculo-skeletal inflammatory illness) these co-crystals would be formulated into a convenient pharmaceutical formulation or a medicament. Accordingly a desirable advantage of a co-crystal of venlafaxine would show improved pharmaceutical properties and features, especially when compared to the free base of venlafaxine or its salts or to celecoxib alone. Thus, the co-crystal of venlafaxine and celecoxib according to the invention should desirably show at least one, preferably more, of the following features:
● to have a very small particle size, e.g. from 300 µm or lower; or
● to be and/or remain essentially free of agglomerates; or
● to be less or not very hygroscopic; or
● to help in formulating controlled release or immediate release formulations; or
● to have a high chemical stability; or
   if given to a patient
● to decrease the inter- and intra-subject variability in blood levels; or
● to show a good absorption rate (e.g. increases in plasma levels or AUC); or
● to show a high maximum plasma concentration (e.g. Cₘₐₓ); or
● to show decreased time to peak drug concentrations in plasma (tₘₐₓ); or
● to show changes in half life of the compound (t_{1/2}), in whichever direction this change is preferably directed.

The association of two active principles in the same crystal exhibits several advantages especially if pharmaceutically formulated. Being linked, they often behave as a single chemical entity, thus facilitating the treatments, formulation, dosage etc. In addition to that, with both venlafaxine and celecoxib being active analgesics these co-crystals are highly useful in the treatment of depression or pain or both symptoms, especially also not losing any activity/weight by the addition of pharmacologically useless counterions as in salts with no active pharmaceutical ingredient. In addition the two active principles are complementing each other in the treatment especially of depression or pain or both in parallel, but possibly also of various other diseases or symptoms. Thus, the co-crystals according to the invention do combine a high number of advantages over the state of the art.

Another advantage is that the association of two active principles into one unique species being formulated together seems to allow for a better Pharmacokinetic/Pharmacodynamic (PKPD) including also a better penetration of the blood-brain barrier, which helps in the treatment of depression and/or pain, especially in the treatment of depression including depression accompanying chronic pain and/or chronic inflammation, more especially in the treatment of depression in patients with a chronic musculo-skeletal inflammatory illness, with the inflammatory illness preferably being selected from osteoarthritis or rheumatoid arthritis.

The medicament or pharmaceutical compositions according to the present invention may be in any form suitable for the application to humans and/or animals, preferably humans including infants, children and adults and can be produced by standard procedures known to those skilled in the art. The medicament of the present invention may for example be administered parenterally, including intramuscular, intraperitoneal, or intravenous injection, transmucosal or sublingual application; or orally, including administration as tablets, pellets, granules, capsules, lozenges, aqueous or oily solutions, suspensions, emulsions, sprays or as reconstituted dry powdered form with a liquid medium.

Typically, the medicaments according to the present invention may contain 1-60 % by weight of one or more of the co-crystals as defined herein and 40-99 % by weight of one or more pharmaceutically acceptable excipients.

The compositions of the present invention may also be administered topically or via a suppository.

The daily dosage for humans and animals (especially adults) may vary depending on factors that have their basis in the respective species or other factors, such as age, sex, weight or degree of illness and so forth. The daily dosage for humans preferably is in the range of 5 to 800 milligrams of venlafaxine or preferably 25 to 500 to be administered during one or several intakes per day e.g, in doses of 25, 37.5, 50, 75, 100, 150 or 400 mg.

The daily dosage for humans preferably is in the range of 5 to 800 milligrams of celecoxib or preferably 50 to 500 milligrams to be administered during one or several (preferably two) intakes per day e.g, in doses of 100 or 200 mg.

The daily dosage for humans of the co-crystal itself preferably is in the range of 12 to 1900 milligrams of the co-crystal or preferably 50 to 1200 milligrams to be administered during one or several (preferably two) intakes per day e.g, in doses of 50, 60, 90, 120, 150, 200 or 500 mg or any dose in between.

A further aspect of the invention relates to the use of a co-crystal according to the invention as described above for the treatment of pain, including chronic pain; or of depression including depression accompanying chronic pain and/or chronic inflammation, preferably for the treatment of depression in patients with a chronic musculo-skeletal inflammatory illness, with the inflammatory illness preferably being selected from osteoarthritis or rheumatoid arthritis. Preferably this use is provided in the form of a medicament or a pharmaceutical composition according to the invention as described above. The pain mentioned above may be chronic pain, but also severe to moderate pain.

A further aspect of the invention relates to the use of a co-crystal according to the invention as described above for the production of a medicament for the treatment of pain, including chronic pain; or of depression, including depression accompanying chronic pain and/or chronic inflammation, preferably for the treatment of depression in patients with a chronic musculo-skeletal inflammatory illness, with the inflammatory illness preferably being selected from osteoarthritis or rheumatoid arthritis. The pain mentioned above may be chronic pain, but also severe to moderate pain.

Another object of the current invention is a method of treatment of pain, including chronic pain; or of depression, including depression accompanying chronic pain and/or chronic inflammation, preferably for the treatment of depression in patients with a chronic musculo-skeletal inflammatory illness, with the inflammatory illness preferably being selected from osteoarthritis or rheumatoid arthritis, by providing to a patient in need thereof a sufficient amount of a co-crystal according to the invention as described above. The pain mentioned above may be chronic pain, but also severe to moderate pain. Preferably the co-crystal according to the invention is provided in physiologically suitable form like e.g. in the form of a medicament or a pharmaceutical composition according to the invention as described above.

The present invention is illustrated below with the help of the following figures and examples. These illustrations are given solely by way of example and do not limit the invention.

### Brief description of the figures:

**Figure 1****:**
   Differential scanning calorimetry (DSC) analysis of a co-crystal of celecoxib ― (rac)-venlafaxine 1:1.
**Figure 2****:**
   Thermogravimetric analysis (TGA) of a co-crystal of celecoxib ― (rac)-venlafaxine 1:1.
Figure 3:
   X-ray powder diffraction (XRPD) pattern of a co-crystal of celecoxib ― (rac)-venlafaxine 1:1.
**Figure 4****:**
   Depiction of the asymmetric unit of the crystal structure of a co-crystal of celecoxib ― (rac)-venlafaxine 1 : 1 (hydrogen atoms having been omitted for clarity), obtained by single crystal X-ray diffraction analysis (SCXRD) showing one molecule of venlafaxine and celecoxib in the unit cell (program used: Mercury 2.2).

### EXAMPLES

### Example 1: celecoxib - (rac)-venlafaxine co-crystal (1:1)

### Processes for the production of celecoxib - (rac)-venlafaxine (1:1) co-crystal:

### Example 1.1 (slow evaporation from methyl isobutyl ketone):

To a vial containing celecoxib (58 mg, 0.15 mmol) and (rac)-venlafaxine (42 mg, 0.15 mmol, 1.0 eq), was added methyl isobutyl ketone (1 mL). The resultant solution was slowly evaporated at room temperature. After complete evaporation, co-crystal celecoxib - *(rac)-*venlafaxine (1:1) was obtained as a white solid (100 mg, quantitative yield).
The experiment was carried out with diethyl ether giving similar results.

### Example 1.2 (wet grinding with ethanol (solvent assisted grinding)):

To a 5 mL stainless steel grinding jar containing celecoxib (58 mg, 0.15 mmol), (rac)-venlafaxine (42 mg, 0.15 mmol, 1.0 eq) and two 7 mm stainless steel grinding balls, one drop of ethanol was added. The reactor was stirred 20 minutes at a rate of 30 Hz (2 x 10 minutes) giving co-crystal celecoxib - (rac)-venlafaxine (1:1) as a white solid (100 mg, quantitative yield).

The experiment was carried out with AcO*i*Bu, methyl isobutyl ketone or toluene giving similar results.

### Example 1.3 (precipitation in acetonitrile):

To an assay tube equipped with magnetic stirrer containing celecoxib (58 mg, 0.15 mmol) and (rac)-venlafaxine (42 mg, 0.15 mmol, 1.0 eq), was added acetonitrile (0.2 mL). The resultant solution was seeded with co-crystal obtained in example 1.1 or 1.2 and a white precipitate was formed in few minutes. The mixture was stirred at room temperature for 5 hours. The solid was filtered with a sintered funnel (porosity 4), washed with cold acetonitrile (0.2 mL) and dried under vacuum at room temperature to give co-crystal celecoxib - *(rac)-*venlafaxine (1:1) as a white solid (22 mg, 22% yield).

### Example 1.4 (crystallization in THF/heptane):

To an assay tube equipped with magnetic stirrer containing celecoxib (58 mg, 0.15 mmol) and (rac)-venlafaxine (42 mg, 0.15 mmol, 1.0 eq), was added THF (0.2 mL). The resultant solution was seeded with obtained in example 1.1 or 1.2 and heptane (0.5 mL) was added before seeding dissolution. The solution was stirred at 0°C for 4 hours. A white precipitate was formed. The solid was filtered with a sintered funnel (porosity 4), washed with cold heptane (0.2 mL) and dried under vacuum at room temperature to give co-crystal celecoxib-(rac)-venlafaxine (1:1) as a white solid (29 mg, 29% yield).

### Example 1.5 (crystallization in methyl isobutyl ketone/heptane (10 g scale)):

To a round-bottom flask equipped with magnetic stirrer containing celecoxib (6.289 g, 16.49 mmol) and (rac)-venlafaxine (5.712 g, 20.59 mmol, 1.2 eq), was added methyl isobutyl ketone (24 mL). The resultant solution was seeded with obtained in example 1.1 to 1.3 and stirred at 0°C. Heptane (60 mL) was added over a 30 minute period and a white precipitate was formed. The resulting slurring was stirred at 0°C for 3 hours. The solid was filtered with a sintered funnel (porosity 4), washed with cold heptane (2 x 15 mL) and dried under vacuum at room temperature to give co-crystal celecoxib - (rac)-venlafaxine (1:1) as a white solid (9.4997 g, 87% yield).

### Characterization of ce+lecoxib - (rac)-venlafaxine (1:1) co-crystal ¹H NMR

Proton nuclear magnetic resonance analyses were recorded in deuterated chloroform (CDCl₃) in a Varian Mercury 400 spectrometer, equipped with a broadband probe ATB 1 H/19F/X of 5 mm. Spectra were acquired dissolving 5-10 mg of sample in 0.6 mL of deuterated solvent.

¹H NMR (CDCl₃, 400 MHz): δ = 7.94-7.87 (m, 2H); 7.51-7.44 (m, 2H); 7.20-7.16 (m, 2H); 7.14-7.09 (d, 2H); 7.07-7.02 (m, 2H); 6.84-6.79 (m, 2H); 6.74 (s, 1H); 3.79 (s, 3H); 3.27 (t, J = 12.5 Hz, 1 H); 2.94 (dd, J = 12.1 Hz and 3.5 Hz, 1 H); 2.38 (s, 3H); 2.32 (s, 6H), 2.30 (dd, J = 12.5 Hz and 3.5 Hz, 1 H); 1.80-1.45 (m, 6H); 1.40-1.24 (m, 2H); 1.00-0.81 (m, 2H).

### IR

FTIR spectra were recorded using a Thermo Nicolet Nexus 870 FT-IR, equipped with a beamsplitter KBr system, a 35mW He-Ne laser as the excitation source and a DTGS KBr detector. The spectra were acquired in 32 scans at a resolution of 4 cm⁻¹.

IR (KBr): v = 3293.8 (m), 2945.7 (m), 2860.9 (m), 2830.3 (m), 1610.3 (m), 1512.5 (s), 1470.0 (s), 1410.0 (m), 1375.0 (m), 1350.3 (m), 1339.7 (s), 1238.3 (s), 1163.1 (s), 1112.1 (m), 1093.5 (m), 1039.8 (m), 974.9 (m), 840.5 (s), 825.4 (m), 805.8 (m) 615.1 (m), 544.8 (m) cm⁻¹.

### DSC

DSC analyses were recorded with a Mettler DSC822^{e}. A sample of 3.3560 mg was weighed into 40 µL aluminium crucible with a pinhole lid and was heated, under nitrogen (50 mL/min), at 10°C/min from 30 to 200°C.

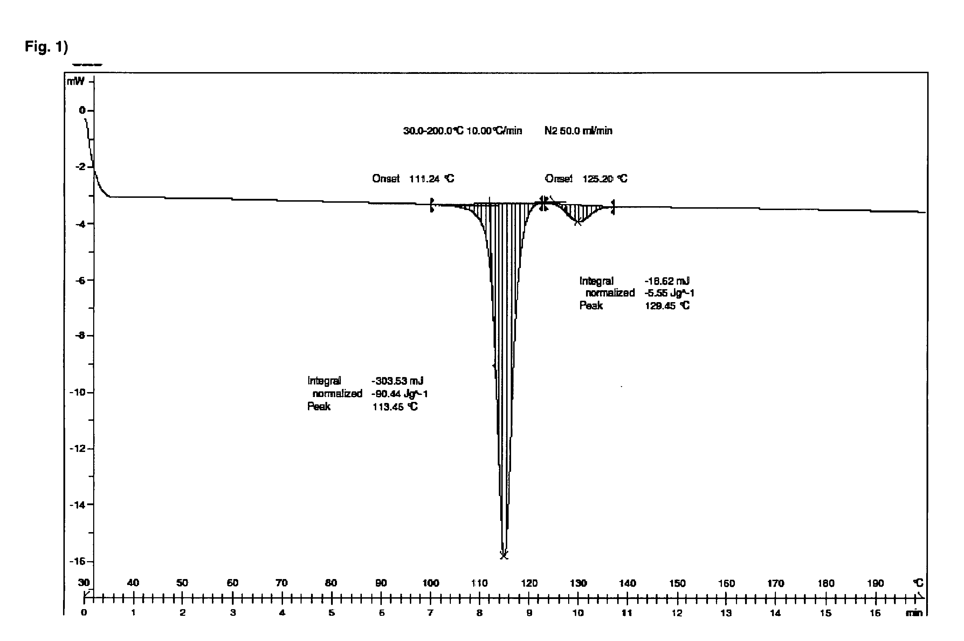

The novel type of crystal of the present invention is characterized in that the endothermic sharp peak corresponding to the melting point has an onset at 111.24 °C (fusion enthalpy - 90.44 J/g), measured by DSC analysis (10 °C/min) (see figure 1).

### TG

Thermogravimetric analyses were recorded in a thermogravimetric analyzer Mettler TGA/SDTA851^{e} A sample of 2.4793 mg was weighed into a 70 µL alumina crucible with a pinhole lid and was heated at 10°C/min from 30 to 200°C, under nitrogen (50 mL/min).

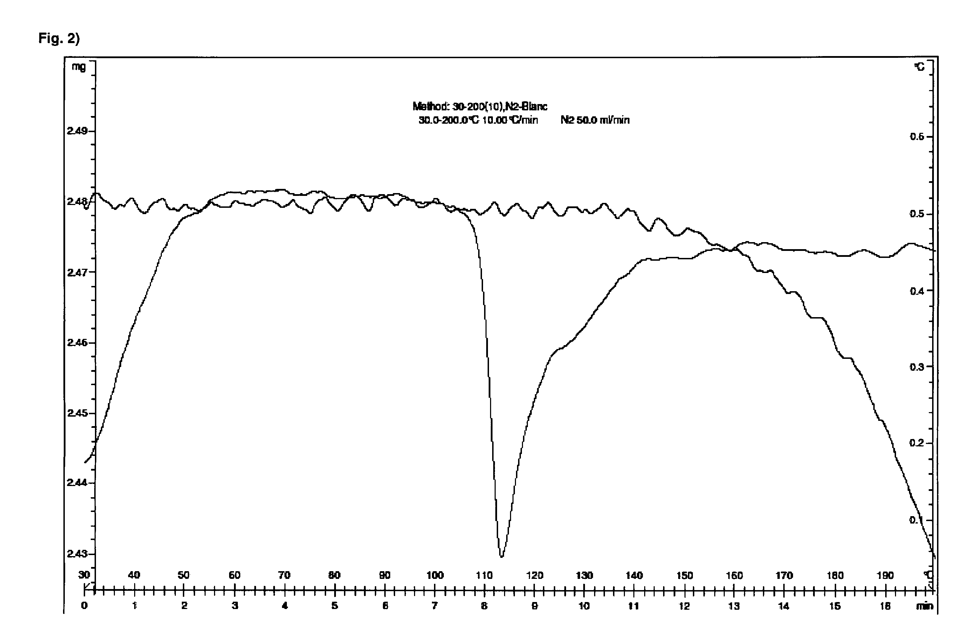

The TG analysis of the crystalline form according to the invention shows no weight loss at temperatures lower than the melting point (see figure 2).

### XRPD

XRPD analysis was performed using a Philips X'Pert diffractometer with Cu K_{α} radiation in Bragg-Brentano geometry. The system is equipped with a monodimensional, real time multiple strip detector. The measurement parameters were as follows: the range of 2θ was 3° to 40° at a scan rate of 8.8° per minute.

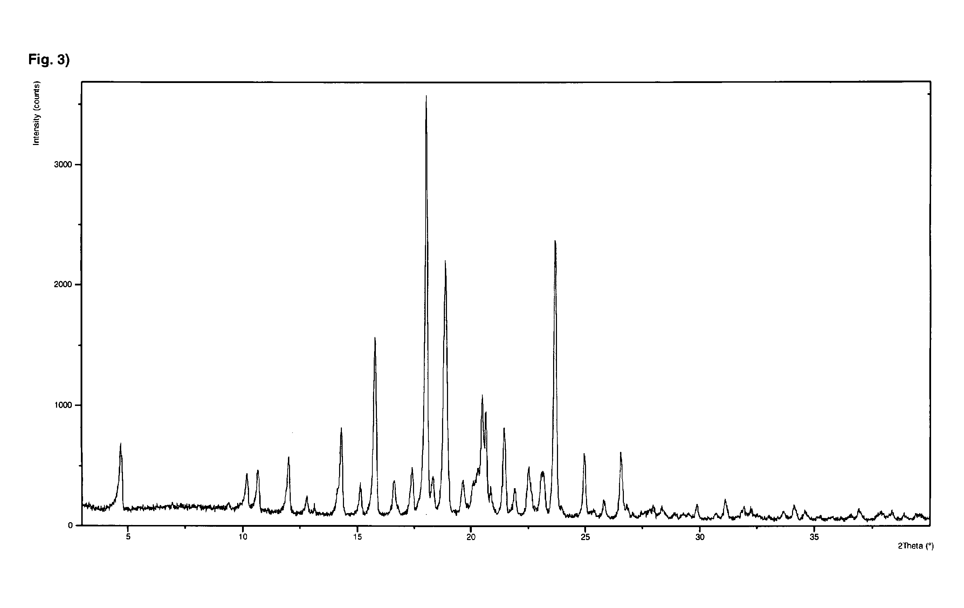

List of selected peaks, measured on a sample from crystallization in methyl isobutyl ketone/heptane, is as follows (only peaks with relative intensity greater than 1 % are indicated) (see figure 3):

| **2θ (°)** | **d (Å)** | **l(%)** | | **2θ (°)** | **d (Å)** | **l (%)** |
|---|---|---|---|---|---|---|
| 4.73 | 18.67 | 13 | | 22.51 | 3.95 | 10 |
| 9.38 | 9.42 | 1 | | 23.19 | 3.84 | 9 |
| 10.18 | 8.69 | 9 | | 23.66 | 3.76 | 64 |
| 10.69 | 8.27 | 9 | | 24.94 | 3.57 | 14 |
| 12.01 | 7.37 | 13 | | 25.34 | 3.51 | 1 |
| 12.81 | 6.91 | 4 | | 25.83 | 3.45 | 4 |
| 14.33 | 6.18 | 20 | | 26.56 | 3.36 | 15 |
| 15.13 | 5.85 | 7 | | 27.99 | 3.19 | 2 |
| 15.81 | 5.61 | 41 | | 28.33 | 3.15 | 2 |
| 16.64 | 5.33 | 8 | | 29.87 | 2.99 | 3 |
| 17.43 | 5.09 | 10 | | 30.72 | 2.91 | 1 |
| 18.03 | 4.92 | 100 | | 31.11 | 2.88 | 4 |
| 18.34 | 4.84 | 8 | | 31.90 | 2.81 | 2 |
| 18.87 | 4.70 | 60 | | 32.24 | 2.78 | 2 |
| 19.63 | 4.52 | 7 | | 33.66 | 2.66 | 2 |
| 20.06 | 4.43 | 6 | | 34.11 | 2.63 | 3 |
| 20.49 | 4.33 | 28 | | 34.57 | 2.59 | 2 |
| 20.65 | 4.30 | 23 | | 36.56 | 2.46 | 1 |
| 20.87 | 4.26 | 6 | | 36.90 | 2.44 | 2 |
| 21.45 | 4.14 | 21 | | 37.90 | 2.37 | 1 |
| 21.93 | 4.05 | 6 | | 38.35 | 2.35 | 2 |

### Single crystal X-ray diffraction

Crystal structure of co-crystal celecoxib ― (rac)-venlafaxine (1:1) has been determined from single crystal X-ray diffraction data. The colourless plate used (0.35 x 0.23 x 0.04 mm) was obtained from the evaporation of a seeded solution in diethyl ether of equimolar amounts of celecoxib and (rac)-venlafaxine.

Analysis was performed at room temperature using a Bruker Smart Apex diffractometer with graphite monochromated Mo Kα radiation equipped with a CCD detector. Data were collected using phi and omega scans (program used: SMART 5.6). No significant decay of standard intensities was observed. Data reduction (Lorentz and polarization corrections) and absorption correction were applied (program used: SAINT 5.0).

The structure was solved with direct methods and least-squares refinement of Fₒ² against all measured intensities was carried out (program used: SHELXTL-NT 6.1). All non-hydrogen atoms were refined with anisotropic displacement parameters. Fluorine atoms were found to be affected by disorder.

Relevant structural data:

| Crystal system | Monoclinic |
|---|---|
| Space group | *P2₁la* |
| a (Å) | 17.7361 (18) |
| b (Å) | 10.1656(10) |
| c (Å) | 20.112(2) |
| β (°) | 111.198(2) |
| Volume (Å³) | 3380.8(6) |
| Z | 4 |
| D calc. (Mg/m³) | 1.29 |
| N. of refl. | 8362 |
| Refl. with l > 2σ(l) | 3200 |
| R (l>2σ(l)) | 0.0776 |

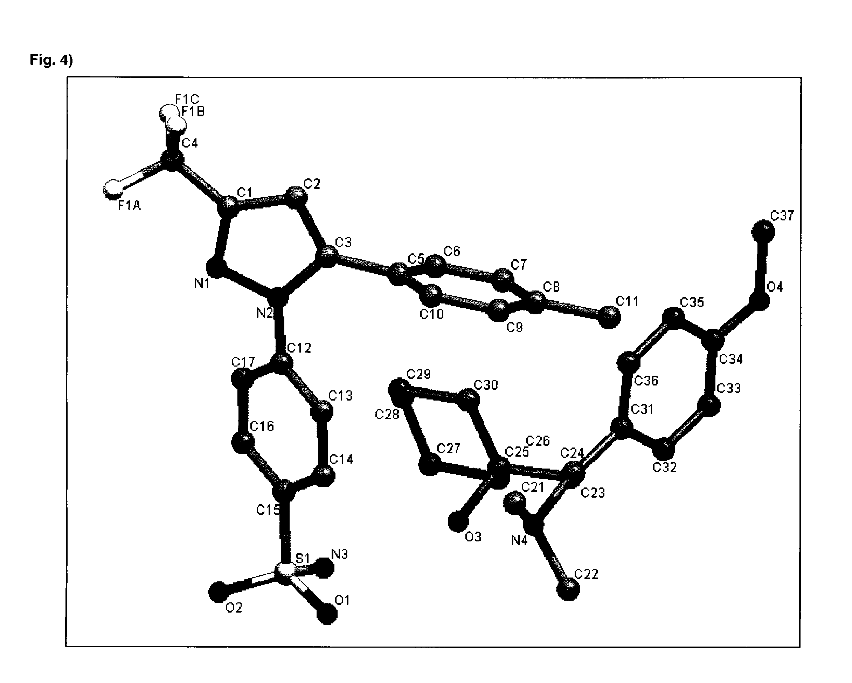

The asymmetric unit of the crystal structure of co-crystal celecoxib ― (rac)-venlafaxine (1:1) is depicted in figure 4 (hydrogen atoms have been omitted for clarity; program used: *Mercury* 2.2).

Simulation of XRPD diffractogram from single crystal data gives an almost identical diagram to the experimental one presented above.

Bibliography.
1. Campbell LC, Clauw DJ, Keefe FJ. (2003). Persistent pain and depression: a biopsychosocial perspective. Biol Psychiatry, 54, 399-409.
2. Kojima M, Kojima T, Suzuki S, Oguchi T, Oba M, Tsuchiya H, Sugiura F, Kanayama Y, Furukawa TA, Tokudome S, Ishiguro N. (2009). Depression, inflammation, and pain in patients with rheumatoid arthritis. Arthritis Rheum. 61, 1018-1024.
3. Tunks ER, Crook J, Weir R. (2008). Epidemiology of chronic pain with psychological comorbidity: prevalence, risk, course, and prognosis. Can J Psychiatry. 53, 224-34.
4. Gatchel RJ, Peng YB, Peters ML, Fuchs PN, Turk DC. (2007). The biopsychosocial approach to chronic pain: scientific advances and future directions. Psychol Bull. 133, 581-624
5. Banks, Sara M.; Kerns, Robert D (1996). Explaining high rates of depression in chronic pain: A diathesis-stress framework. Psychol Bull. 119, 95-110.
6. MacDonald, G., Leary, MR., (2005). Why Does Social Exclusion Hurt? The Relationship Between Social and Physical Pain. Psychol Bull. 131, 202-223.
7. Millan MJ. (1999). The induction of pain: an integrative review. Prog Neurobiol. 57, 1-164.
8. Arnold LM, Meyers AL, Sunderajan P, Montano CB, Kass E, Trivedi M, Wohlreich MM. (2008). The effect of pain on outcomes in a trial of duloxetine treatment of major depressive disorder. Ann Clin Psychiatry, 20, 187-93.
9. Goldenberg MM. (1999). Celecoxib, a selective cyclooxygenase-2 inhibitor for the treatment of rheumatoid arthritis and osteoarthritis. Clin Ther. 21, 1497-513

## Claims

1. A co-crystal comprising venlafaxine either as a free base or as its physiologically acceptable salt and celecoxib.

2. The co-crystal according to claim 1 wherein the venlafaxine is selected from (rac)-venlafaxine, (-)-venlafaxine or (+)-venlafaxine, preferably is (rac)-venlafaxine.

3. The co-crystal according to any of claims 1 or 2, wherein the molecular ratio between celecoxib and venlafaxine is between 3:1 and 1:3, preferably is between 2:1 and 1:2, most preferably is 1:1.

4. The co-crystal according to any of claims 1 to 3, wherein the molecular ratio between celecoxib and (rac)-venlafaxine is 1:1.

5. Co-crystal according to claim 4, **characterized in that** the endothermic sharp peak of the co-crystal corresponding to the melting point has an onset at 111°C.

6. Co-crystal according to any of claims 4 or 5, **characterized in that** it shows an X-Ray powder diffraction pattern with peaks [2θ] at 4.73, 12.01, 14.33, 15.81, 17.43, 18.03, 18.87, 20.49, 20.65, 21.45, 22.51, 23.66, 24.94, and 26.56 with the 2θ values being obtained using copper radiation (Cu_{Kα1}) 1.54060Å).

7. Co-crystal according to claim 6, **characterized in that** it shows a X-Ray powder diffraction pattern peaks [2θ] at 4.73, 9.38, 10.18, 10.69, 12.01, 12.81, 14.33, 15.13, 15.81, 16.64, 17.43, 18.03, 18.34, 18.87, 19.63, 20.06, 20.49, 20.65, 20.87, 21.45, 21.93, 22.51, 23.19, 23.66, 24.94, 25.34, 25.83, 26.56, 27.99, 28.33, 29.87, 30.72, 31.11, 31.90, 32.24, 33.66, 34.11, 34.57, 36.56, 36.90, 37.90 and 38.35. The 2θ values were obtained using copper radiation (C_{UKα1} 1.54060Å).

8. Co-crystal according to claim 4 to 7, **characterized in that** it has a monoclinic unit cell with the following dimensions:
a = 17.74(18) Å
b=10.17(10) Å
c = 20.11 (2) Å
β = 111.20(2)°

9. Process for the production of a co-crystal according to claims 1 to 8 comprising the steps of:
(a) adding venlafaxine and celecoxib to a container;
(b) adding a first organic solvent;
(c) optionally adding a second organic solvent;
(d) optionally removing the first organic solvent;
(e) optionally filtering-off the resulting co-crystals
(f) optionally drying the resulting co-crystals at ambient temperature under vacuum.

10. Pharmaceutical composition comprising the co-crystal according to any claims 1 to 8 and optionally one or more pharmaceutical ingredients.

11. Pharmaceutical composition according to claim 10 for the treatment of depression including depression accompanying chronic pain and/or chronic inflammation, preferably for the treatment of depression in patients with a chronic musculo-skeletal inflammatory illness, with the inflammatory illness preferably being selected from osteoarthritis or rheumatoid arthritis.

12. Use of a co-crystal according to any one of claims 1 to 8 for production of a medicament for the treatment of depression including depression accompanying chronic pain and/or chronic inflammation, preferably for the treatment of depression in patients with a chronic musculo-skeletal inflammatory illness, with the inflammatory illness preferably being selected from osteoarthritis or rheumatoid arthritis.
